# EUROPÄISCHE PATENTSCHRIFT

(11) **EP 1 251 835 B1**
(45) Veröffentlichungstag und Bekanntmachung des Hinweises auf die Patenterteilung: **13.08.2008**
(21) Anmeldenummer: 01916954.9
(22) Anmeldetag: 26.01.2001
(51) Int. Cl.: A61K 9/50, A61K 48/00, C12N 15/87

(54) **VERSCHLIESSEN VON BAKTERIENGHOSTS**
CLOSURE OF BACTERIAL GHOSTS
FERMETURE D'HOTES BACTERIENS

(30) Priorität: 26.01.2000 DE 10003241
(43) Veröffentlichungstag der Anmeldung: 30.10.2002
(73) Patentinhaber: Prof. Dr. Lubitz, Werner, 3420 Klosterneuburg/Kritzendorf (AT)
(72) Erfinder: LUBITZ, Werner, A-3420 Klosterneuburg/Kritzendorf (AT); PAUKNER, Susanne, A-3500 Krems (AT)
(74) Vertreter: Weiss, Wolfgang
(86) Internationale Anmeldenummer: PCT/EP2001/000864
(87) Internationale Veröffentlichungsnummer: WO 2001/054672

(56) Entgegenhaltungen:
- HUTER V ET AL.: "Bacterial ghosts as drug carrier and targeting vehicles." JOURNAL OF CONTROLLED RELEASE, Bd. 61, Nr. 1-2, 27. August 1999 (1999-08-27), Seiten 51-63, XP000907214 ISSN: 0168-3659
- LUBITZ W ET AL.: "Extended recombinant bacterial ghost system." JOURNAL OF BIOTECHNOLOGY, Bd. 73, Nr. 2-3, 20. August 1999 (1999-08-20), Seiten 261-273, XP004180188 ISSN: 0168-1656

## Beschreibung

Die Erfindung betrifft ein Verfahren zur Herstellung von verschlossenen Bakterienghosts mittels Vesikel-Membran-Fusion und die auf diese Weise erhältlichen Bakterienghosts. In die verschlossenen Bakterienghosts können Wirkstoffe, z.B. genetisches Material, Zellkomponenten, pharmazeutische und landwirtschaftliche Wirkstoffe sowie Markierungssubstanzen oder Farbstoffe verpackt werden. Bei Verpackung von genetischem Material und gegebenenfalls anderen Komponenten, z. B. Komponenten des Cytoplasmas, in die Bakterienghosts können metabolische Funktionen und gegebenenfalls die Proliferationsfähigkeit der Zellen wieder hergestellt werden. Die verschlossenen Ghosts können in der Medizin, im Argrarbereich und in der Biotechnologie eingesetzt werden.

Leere Bakterienhüllen, sogenannte Bakterienghosts, können durch kontrollierte, heterologe Expression eines Gens, das eine partielle Lyse der Zellmembran bewirkt, in gram-negativen Bakterien hergestellt werden (EP-A-0 291 021). Ein Beispiel für ein derartiges lytisches Gen ist das Gen E des Bakteriophagen PhiX174, das für ein Polypeptid kodiert, welches in den Zellwandkomplex gram-negativer Bakterien inseriert und durch die Oligomerisierung zur Ausbildung einer transmembranen Tunnelstruktur durch die innere und äußere Membran führt. Der innere Durchmesser dieser Tunnelstruktur kann je nach Lysebedingungen 40 bis 200 nm oder 500 bis 1000 nm betragen. Durch diesen Tunnel wird das cytoplasmatische Material der Zelle freigesetzt und eine leere Zellhülle mit intakter Morphologie zurückgelassen. Die Verwendung von Bakterienghosts als Totimpfstoffe oder Adjuvanzien sowie die Herstellung rekombinanter Bakterienghosts, die in ihrer Membran heterologe Oberflächenproteine tragen, wird in WO 91/13555 und WO 93/01791 beschrieben.

Weiterhin können Ghosts auch aus Gram-positiven Bakterien durch Verwendung eines chimären E-L-Lysegens hergestellt werden (US-A-5,075,223).

In DE 199 09 770.4 wird vorgeschlagen, Wirkstoffe in Bakterienghosts zu verpacken. Aufgrund der Löcher in den Ghostmembranen ist eine Retention der Wirkstoffe innerhalb der Ghosts oftmals nur durch aufwendige Maßnahmen erreichbar.

Huter et al. (Journal of Controlled Release, 61, 1999, 51-63) offenbaren leere Bakterienhüllen als Träger für Arzneimittel und Impfstoffe, wobei die leeren Bakterienhüllen große Öffnungen haben.

Überraschenderweise wurde festgestellt, dass sich Bakterienghosts mittels einer Vesikel-Membran-Fusion unter geeigneten Bedingungen wieder verschließen lassen, wobei um den Innenraum der Zellen eine kontinuierliche Membran gebildet wird. Hierzu werden kompetente Bakterienghosts mit Membran-Lipidvesikeln unter Bedingungen in Kontakt gebracht, bei denen eine Fusion zwischen der Membran der Bakterienghosts und der Membran der Membran-Lipidvesikel erfolgt.

Besonders bevorzugt stammen die Ghosts von gram-negativen Bakterien, die z.B. ausgewählt werden aus Escherichia coli, Klebsiella, Salmonella, Enterobacter, Pseudomonas, Vibrio, Actinobacillus, Haemophillus, Pasteurella, Bordetella, Helicobacter, Francisella, Brambamella, Erwinia, Pantoea, Streptomyces, Frankia, Serratia, Agrobacterium, Azotobacter, Bradyrhizobium, Burkholderia, Rhizobium, Rhizomonas und Sphingomonas. Besonders bevorzugte Beispiele für gram-positive Bakterien sind Staphylococcus, Streptococcus und Bacillus.

Weiterhin können auch Ghosts eingesetzt werden, die von rekombinanten Bakterien stammen und heterologe Membranproteine enthalten. Diese Ghosts mit modifizierten Hüllen sind insbesondere für eine human- oder veterinärmedizinische Verabreichung bedeutsam, die ein Targeting, d.h. einen Transport der Ghosts an Zielzellen oder Zielgewebe erfordert. Hierzu können modifizierte Ghosts eingesetzt werden, die targetspezifische Oberflächenmoleküle an ihrer Membranaußenseite tragen. Die Einführung dieser targetspezifischen Oberflächenmarker wie etwa Zucker, z.B. Mannose oder Fucose, oder Proteine wie Invasin aus Yersinien oder Invasinderivaten, kann durch rekombinante Expression entsprechender membranständiger Fusionspolypeptide in der Bakterienzelle vor der Lyse oder/und durch Anlagerung an die Membran mittels eines geeigneten Rezeptorsystems, z.B. Streptavidin/Biotin, erfolgen.

Um eine effiziente Herstellung von verschlossenen Bakterienghosts zu ermöglichen, ist es zunächst zweckmäßig kompetente Bakterienghosts bereitzustellen. Hierzu werden Bakterienghosts durch Inkontaktbringen mit zweiwertigen Metallkationen und anschließender Inkubation bei einer tiefen Temperatur kompetent gemacht. Als mehrwertige Metallkationen werden vorzugsweise Erdalkalimetallionen, insbesondere Calciumionen verwendet. Das Inkontaktbringen kann beispielweise durch Waschen pelletierter Ghosts mit einer die Metallionen enthaltenden wässrigen Lösung erfolgen, welche die Metallionen in einer Konzentration z.B. von 50 bis 200 mmol/l enthält. Anschließend werden die Bakterienghosts vorzugsweise bei einer Temperatur von 0 bis 5°C für eine ausreichende Zeitdauer inkubiert, z.B. indem sie auf Eis gestellt werden. Die resultierenden kompetenten Ghosts können unmittelbar oder nach Einfrieren und anschließendem Auftauen für die Membranfusion eingesetzt werden. Es können jedoch auch Bakterienghosts ohne vorherige Behandlung verwendet werden.

Die Membranfusion umfasst eine Fusion zwischen der Membran der Bakterienghosts und der Membran von Lipidvesikeln. Die Lipidvesikel können aus natürlichen oder synthetischen Quellen stammen und enthalten vorzugsweise eine Lipid-Doppelschicht, die Phospholipide wie etwa Phosphatidylethanolamin enthält. Beispielsweise können Vesikel eingesetzt werden, die bei Homogenisierung von Zellen, insbesondere Bakterienzellen, z.B. durch Ultraschall oder in einer Frenchpress entstehen. Andererseits können auch synthetische Lipidvesikel wie Liposomen eingesetzt werden.

Weiterhin als Lipidvesikel geeignet sind membranumhüllte Viren wie etwa Pox-, Chordopox-, Herpes-, Hepadnaviridae (DNA-Viren) sowie Corona-, Paramyxo-, Bunya-, Orthomxyxo-, Arena-, Toga-, Flavi-, Retro-, Rhabdoviridae (RNA-Viren). Selbstverständlich können auch Kombinationen der genannten Lipidvesikel eingesetzt werden.

Die Fusion der Bakterienghosts mit den Lipidvesikeln erfolgt vorzugsweise unter Bedingungen, bei denen sowohl die Membran der Bakterienghosts als auch die Membran der Lipidvesikel in einem flüssigen Zustand ist, z.B. bei einer Temperatur von ≥ 30°C, z.B. 37°C. Um eine Fusion zu erzielen, werden die Membranen in engen Kontakt gebracht, so dass elektrostatische Abstoßungskräfte zwischen den Bakterienghosts und den Lipidvesikeln überwunden und die Membran in den Ausgangsmaterialien destabilisiert werden. Solche Bedingungen werden beispielsweise während einer Ultrazentrifugation erreicht. Weitere Methoden zur Überwindung elektrostatischer Abstoßungskräfte sind beispielsweise in Molecular Biology of Membranes, Structure and Function, Howard R. Petty, Kaptiel 8: Seiten 297 - 345, beschrieben und umfassen die Verwendung chemischer Fusogene wie Polyethylenglykol, Glycerin, DMSO oder/und Polyhistidin, welche eine Verringerung des Oberflächenpotentials und somit eine Verringerung der elektrostatischen Abstoßung bewirken. Bei Verwendung von phospholipidhaltigen Vesikeln kann die Fusion durch Zugabe von Calcium verbessert werden. Bei Verwendung von viralen Lipidvesikeln kann die Fusion durch das Vorhandensein von Proteinen in der Virenmembran, z.B. Influenza-Hämagglutinin, Sendai-F-Protein, Semliki Forest-Spike-Glykoprotein, Vesicular Stomatitis Virus (VSV)-VSVG-Protein etc. verbessert werden. Außerdem können auch Elektrofusionstechniken, wie in Methods in Molecular Biology, Vol. 48: Animal Cell Electroporation and Electrofusion Protocols; Kenneth L. White, Kapitel 23, Seiten 283 - 293 eingesetzt werden.

Besonders bevorzugt erfolgt die Fusion zwischen den Membranen der Bakterienghosts und der Lipidvesikel in Gegenwart von Hilfsmitteln, z.B. zweiwertigen Metallkationen, insbesondere Calciumionen oder/und organischen Aggregationshilfsmitteln, beispielweise Glycerin, Polyethylenglykol, Dimethylsulfoxid, Polyhistidin oder Kombinationen davon erfolgt. Die Konzentration zweiwertiger Metallionen liegt vorzugsweise im Bereich von 10 mM bis 25 mM. Die Konzentration der organischen Aggregationsmittel liegt vorzugsweise im Bereich von 10 bis 25 % (w/v).

Ein wichtiger Aspekt der Erfindung beinhaltet das Verpacken von Wirkstoffen in den geschlossenen Bakterienghosts. Die Wirkstoffe kann jeder beliebige, in das Innere der Bakterienghosts transportierbare und dort gegebenenfalls immobilisierbare Wirkstoff sein. Beispiele für Wirkstoffe sind pharmakologisch wirksame Substanzen, Markierungssubstanzen, landwirtschaftlich wirksame Substanzen, Farbstoffe, aber auch genetisches Material und Zellkomponenten, z.B. Zellextrakte, Bestandteile von Zellextrakten oder Zellorganellen wie Ribosomen sein.

Die Verpackung der Wirkstoffe kann auf unterschiedliche Art und Weise erfolgen. So können die Wirkstoffe vor dem Verpacken in die Ghosts eingebracht werden und dort gegebenenfalls immobilisiert werden. Weiterhin können die Wirkstoffe auch in gelöster Form im Verpackungsmedium vorhanden sein. Darüber hinaus ist es möglich, die Wirkstoffe in die zur Fusion mit den Ghosts verwendeten Lipidvesikel zu verpacken. Verfahren zum Verpacken von Wirkstoffen in Lipidvesikel sind bekannt, siehe z.B. J. Treat et al., Liposomes in the Therapy of Infections, Diseases and Cancer, G. Lopez-Berestein und I.J. Fidler, Eds. (Liss, New York, 1989), S. 353 - 365, (Doxorubicin); G. Lopez-Berestein ibid., S. 317 - 327 (Amphotericin B); E.S. Kleineman et al., Cancer Res. 49: 4665 (1989), G. Poste et al., ibid. 42, 1412 (1982); G.R. Alving et al., Vaccine 4, 166 (1986) (Vakzine); A.G. Allison und G. Gregoriadis, Nature 252: 252 (1974) (Vakzine); V.V. Ranade, J. Clin. Pharmacol. (1989) 29: 685 - 694; S.S. Davis, Drugs Exp. Clin. Res. (1985) 11: 633-640; T.M. Allen, Drugs (1998) 56: 747 - 756; P.P. Speiser, Methods Find Exp. Clin. Pharmacol. (1991) 13: 337 - 342; R. Singh und S.P. Vyas, J. Dermatol. Sci. (1996) 13: 107 - 111; P.N. Shek et al., J. Drug Target (1994) 2: 431 - 442; Z. Pavelic et al., Eur. J. Pharm. Sci. (1999) 8: 345- 351; J. M. Sollovitz et al., Vet. Res. (1998) 29: 409 - 430 sowie die darin zitierten Literaturstellen.

Beispiele für pharmakologisch wirksame Substanzen sind Polypeptide wie etwa Antikörper, therapeutisch wirksame Polypeptide wie Zytokine, Interferone, Chemokine etc., Enzyme und immunogene Polypeptide oder Peptide. Ein weiteres Beispiel für Wirkstoffe sind Nukleinsäuren, z.B. DNA oder/und RNA, insbesondere therapeutische Nukleinsäuren, z.B. Nukleinsäuren für die Gentherapie, die vorzugsweise in Form eines chromosomal integrierbaren Vektors vorliegen, oder Nukleinsäuren für eine Nukleinsäure-Vakzinierung, Antisense-Nukleinsäuren oder Ribozyme. Noch weitere Beispiele für Wirkstoffe sind niedermolekulare Wirksubstanzen, Peptide, Hormone, Antibiotika, Antitumormittel, Steroide, Immunmodulatoren etc. Die Wirkstoffe können in den Bakterienghosts in gelöster Form, als Suspensionen oder/und als Emulsionen gegebenenfalls in Kombination mit geeigneten Träger- oder/und Hilfsstoffen vorliegen. Weiterhin können die Wirkstoffe auch diagnostische Markierungssubstanzen, z.B. fluoreszierende Substanzen, Farbstoffe oder Röntgenkontrastmittel sein.

Auch nichtmedizinische Wirkstoffe können in Ghosts verpackt werden, z.B. Wirkstoffe aus dem Agrarbereich wie Insektizide, Herbizide, Mittel gegen Nematoden, Enzyme zur Bodenverbesserung, Dünger, Wachstumsförderer, wasserbindende Proteine zur besseren Durchfeuchtung oder Wasserbindung in der Atmosphäre. Andere Anwendungen sind die Verpackung von Farbstoffen für die Druckindustrie, z.B. fälschungssichere Tinten mit immunologischer Nachweismöglichkeit und die Verpackung von Vitaminen oder Probiotika für die Lebensmittelindustrie. Ebenso möglich ist die Verpackung von kosmetischen Mitteln oder von Stoffen wie Salzen oder anderen ionischen Substanzen.

Der Wirkstoff kann in den Bakterienghosts in immobilisierter Form vorliegen. Die Immobilisierung des Wirkstoffs kann mittels kovalenter oder nichtkovalenter Wechselwirkungen, z.B. elektrostatischer Wechselwirkungen, hochaffiner biologischer Wechselwirkungen, durch mechanische Retention oder eine Kombination von zwei oder mehreren der genannten Möglichkeiten erfolgen.

In einer bevorzugten Ausführungsform der Erfindung erfolgt die Immobilisierung des Wirkstoffs über direkte oder indirekte Wechselwirkungen mit einem Rezeptor, der auf der Membraninnenseite, z.B. der Innenseite der Zytoplasmamembran, der Ghosts, als integraler Membranbestandteil oder als nichtintegraler Membranbestandteil auf der Membran verankert lokalisiert ist. Der Rezeptor kann beispielsweise ein heterologes Polypeptid sein, das über einen oder mehrere Membrananker in die zytoplasmatische Membran der Ghosts integriert ist und durch heterologe Expression entsprechender Fusionsproteine, die mindestens eine Membranankerdomäne sowie mindestens eine Rezeptordomäne enthalten, in den Bakterienzellen vor der Lyse zu den Ghosts erzeugt wird. Bevorzugte Beispiele für Rezeptordomänen sind Avidin oder Streptavidin, die zur Ausbildung hochaffiner Bindungen mit Biotin oder Biotinanaloga in der Lage sind. Besonders bevorzugt ist Streptavidin. Die Verankerung von Streptavidin in Bakterienghosts erfolgt vorzugsweise durch rekombinante Expression eines Streptavidin-Fusionsproteins mit einem C-terminalen Membrananker in der zytoplasmatischen Membran von Bakterien vor der zur Bildung von Ghosts führenden Lyse. Darüber hinaus sind auch andere Rezeptordomänen geeignet, z.B. Antikörperbindungsstellen, Lectine, DNA-Bindungsproteine, Chaperone, Enzyme etc., die mit einem Bindepartner eine hochaffine Bindung eingehen können.

Alternativ kann der Wirkstoff innerhalb des Ghosts auch in freier Form vorliegen, da nach dem Verschließen der Ghosts ein Verlust des Wirkstoffs durch die Membran hindurch im wesentlichen ausgeschlossen ist.

In einer besonders bevorzugten Ausführungsform der Erfindung werden genetisches Material und gegebenenfalls cytoplasmatische Komponenten, z.B. Organellen wie Ribosomen, tRNA, RNA, ATP, Aminosäuren, Nukleotide, translatorische und transkriptionelle Proteine bzw. Enzyme, DNA-Replikationsenzyme und Faktoren, diverse Ionen und Spurenelemente in den Bakterienghost verpackt und anschließend eine Membranfusion durchgeführt. Auf diese Weise lassen sich funktionsfähige Zellen rekonstituieren, sofern das genetische Material eine zur Wieder-herstellung metabolischer Funktionen der verschlossenen Zelle ausreichende Informationen enthält. In einer besonders bevorzugt Ausführungsform enthält das genetische Material sogar eine zur Wiederherstellung der Proliferationsfähigkeit des verschlossenen Bakterienghosts ausreichende Information, d.h. es wird eine Zelle erzeugt, die wieder in der Lage ist, sich zu vermehren. Das hierbei in den Ghost eingeführte genetische Material umfasst vorzugsweise ein partiell, z.B. mindestens 50 % und besonders bevorzugt mindestens 90 % deletiertes bakterielles Genom oder/und extrachromosomales genetisches Material, wie Plasmide, oder virale Genome, welche von der gleichen Spezies wie die Bakterienhülle stammen können. Selbstverständlich können auch Genome eingesetzt werden, die gegebenenfalls partiell deletiertes genetisches Material von anderen Organismen, insbesondere von anderen Bakterienspezies, enthalten. Auch "rekombinante" Genome, die genetisches Material aus mehreren verschiedenen Spezies enthalten, können verpackt werden. Alternativ können auch artifizielle Genome oder Chromosomen in den Ghost eingeführt werden.

Ein weiterer Gegenstand ist somit ein verschlossener Bakterienghost, der durch das zuvor beschriebene Verfahren erhältlich ist. Der verschlossene Bakterienghost enthält eine intakte Membran, d.h. eine das Innere des Ghosts von der Umgebung trennende kontinuierliche Lipidschicht, vorzugsweise eine kontinuierliche Lipid-Doppelschicht. Die verschlossenen Bakterienghosts können verkapselte Wirkstoffe enthalten, metabolische Funktionen aufweisen oder/und die Fähigkeit zur Proliferation besitzen.

Die Herstellung der erfindungsgemäßen verschlossenen und gegebenenfalls mit Wirkstoffen beladenen Ghosts umfaßt zunächst die Herstellung der Bakterienghosts nach bekannten Methoden, z.B. durch Transformation der Bakterienzelle mit einem Lysegen, vorzugsweise dem Gen E des Phagen PhiX174 oder dem chimären E-L-Gen. Die Expression des Lysegens in der Bakterienzelle erfolgt vorzugsweise durch eine regulierbare Expressionskontrollsequenz, z.B. durch das Temperatur-regulierbare Promotor/Repressor-System λ-pR/cl857. Bei diesem Expressionskontrollsystem werden die transformierten Bakterien bei Temperaturen unterhalb 30°C kultiviert. Durch Temperaturerhöhung, vorzugsweise auf ≥40°C wird der thermosensitive λc1857 Repressor inaktiviert und das Lysegen exprimiert, was zur Ausbildung einer transmembranen Tunnelstruktur in der Zellhülle führt, wobei die Zellen innerhalb von wenigen Minuten lysiert werden. Durch mutierte A-Promotor/Operator-Systeme ist eine Anzucht der Bakterien auch bei höheren oder tieferen Temperaturen, z.B. 37°C möglich (W098/07874). Die Bakterienghosts können dann durch Zentrifugation geerntet und nach Waschen und gegebenenfalls Gefriertrocknen der oben beschriebenen Membranfusionsprozedur unterzogen werden. Falls die Ghosts zuvor mit Wirkstoffen beladen werden sollen, können sie mit einer den zu verpackenden Wirkstoff enthaltenden Lösung oder/und Suspension in Kontakt gebracht werden, unter Bedingungen, die das Eindringen ausreichender Wirkstoffmengen in die Bakterienghosts erlauben. Sofern erforderlich, werden darüber hinaus Rezeptorsubstanzen zugesetzt, die eine Immobilisierung der Wirkstoffmoleküle an der Membraninnenseite der Ghosts ermöglichen. Die Zugabe der Rezeptormoleküle kann vor, gleichzeitig oder nach dem Inkontaktbringen der Ghosts mit dem zu verpackenden Wirkstoff erfolgen. Alternativ oder/und zusätzlich können freie Wirkstoffe dem Verpackungsmedium zugesetzt oder/und in den Lipidvesikeln verkapselt werden.

Eine Ausführungsform der Erfindung ist der Einsatz der verschlossenen und gegebenenfalls Wirkstoffe enthaltenden Ghosts für medizinische Zwecke. Die Verabreichung von Wirkstoffen, z.B. pharmakologischen Wirkstoffen, Antigenen, Antikörpern oder Nukleinsäuren, über Ghosts ist zur Prävention oder/und zur Bekämpfung aller Arten von Erkrankungen geeignet, z.B. zur Bekämpfung von durch Pathogene wie Viren, Bakterien, Parasiten oder Pilzen hervorgerufenen Erkrankungen, oder zur Prävention oder/und zur Bekämpfung von Tumor- oder Autoimmunerkrankungen oder zur Gentherapie. Als Wirkstoff wird dabei eine gegen die jeweilige Erkrankung wirksame Substanz verwendet, die nach Transport und gegebenenfalls Internalisierung in der Zielzelle ihre physiologische Wirkung hervorrufen. Die vorliegende Erfindung ermöglicht auch die Verabreichung von Wirkstoffkombinationen, d.h. die Ghosts können mehrere verschiedene Wirkstoffe enthalten oder es können Mischungen von Ghosts mit jeweils verschiedenen Wirkstoffen verwendet werden. Weiterhin kann die Verabreichung von Wirkstoffen über Ghosts auch für diagnostische Zwecke (Imaging) eingesetzt werden.

Eine besonders bevorzugte Anwendung ist der Einsatz von Bakterienghosts als Träger- und Targetingvehikel für die Gentherapie. Durch Verpackung von Nukleinsäuren wie DNA oder RNA in Ghosts kann die mangelnde Spezifität bestehender Nukleinsäure-Vehikel wie z.B. Liposomen entscheidend verbessert werden. Der Vorteil von Bakterienghosts als Trägervehikel besteht weiterhin darin, dass sie eine hohe Kapazität für die Beladung mit Nukleinsäuren besitzen. Sie sind zudem als Vektoren ungefährlich, da sie keine lebenden Zellhüllen darstellen.

Noch eine besonders bevorzugte Anwendung ist der Einsatz von Bakterienghosts zur Herstellung einer Nukleinsäure-Vakzine, insbesondere zur Herstellung einer DNA-Vakzine, und der Einsatz von Bakterienghosts als Träger- oder/und Targetingvehikel für eine Nukleinsäure-Vakzine, insbesondere für eine DNA-Vakzine.

Bakterienghosts als Träger- oder Targetingvehikel zur Nukleinsäure-Vakzinierung führen zur Bildung einer wirkungsvollen und langandauerenden spezifischen Immunantwort. Die Nukleinsäuren enthaltenden Bakterienghosts werden von primären Antigenpräsentierenden Zellen (APC), wie etwa dendritischen Zellen und Makrophagen durch spezifische Rezeptoren aufgenommen und in antigene Peptide fragmentiert. Zusätzlich wird mit hoher Effizienz das Antigen, welches durch die verpackte DNA-Sequenz kodiert wird, in den APC exprimiert. Dies hat zur Folge, dass das Antigen auf der Oberfläche der APC im Kontext mit MHC-I oder/und MHC-II Strukturen den T-Lymphozyten präsentiert wird und eine Immunantwort induzieren kann. Untersuchungen ergaben in diesem Zusammenhang, dass Antigenprozessierungen und Präsentation durch MHC-I und II-Komplexe erfolgen, wobei eine humorale und zelluläre Immunantwort induziert wird, wie sie auch bei bakteriellen Infektionen mit Lebendkeimen beobachtet wird.
Die in die Bakterienghosts verpackte Nukleinsäure ist vorzugsweise in einer im Empfängerorganismus nicht replizierbaren Form. Sie enthält eine Sequenz, die für das in der Zielzelle zu exprimierende Antigen kodiert, in expressionsfähiger Form, d.h. in operativer Verknüpfung mit in der Zielzelle aktiven Expressionskontrollsequenzen wie etwa Promotoren und gegebenenfalls Enhancern, um eine hohe Genexpression zu erlauben, Polyadenylierungssequenzen, um eine korrekte Termination der transkribierten mRNA zu gewährleisten, oder/und Translationsinitiations-Sequenzen, um eine hohe Proteinproduktion zu ermöglichen. Weiterhin können die Nukleinsäuren einen bakteriellen Replikationsursprung, welcher die Amplifikation großer Nukleinsäuremengen in Bakterien wie etwa E.coli erlaubt, ein prokaryontisches Selektionsmarkergen, z.B. für eine Antibiotikaresistenz, ein Reportergen, das eine einfache Bestimmung der Expressionshöhe ermöglicht, z.B. das GFP-Gen oder/und immunmodulatorische Sequenzen enthalten.

Vorzugsweise ist die Nukleinsäure eine DNA, besonders bevorzugt eine Plasmid-DNA, die in zirkulärer oder/und in linearer Form vorliegen kann. Es ist jedoch auch die Verwendung von RNA-Vakzinen oder Vakzinen auf Basis von Nukleinsäureanaloga, die transkribierbar sind, aber eine erhöhte physiologische Stabilität aufweisen, denkbar.

Der die Expression der Antigen-kodierenden Sequenz antreibende Promotor ist vorzugsweise ein starker viraler Promotor/Enhancer, z.B. der Rous Sarcoma Virus (RSV)-Promotor/Enhancer, der Murine Leukemia Virus (MLV)-Promotor/Enhancer, der SV40-Promotor/Enhancer und besonders bevorzugt der Cytomegalovirus (CMV)-Promotor/Enhancer. Als Transkriptions-terminatoren können die Polyadenylierungssequenzen aus SV40 oder aus dem Rinderwachstumshormongen, vorzugsweise jedoch aus dem Kaninchen-β-Globin-Gen verwendet werden.

Als Antigen wird dabei ein mit der jeweiligen Erkrankung assoziiertes Polypeptid oder ein Peptidfragment davon verwendet, das nach Expression in der Zielzelle eine Immunantwort induziert. Die vorliegende Erfindung ermöglicht auch die Verabreichung von Kombinationsvakzinen, d.h. die Ghosts können mehrere verschiedene Antigen-kodierende Nukleinsäuren enthalten, die z.B. vom selben Erreger oder von unterschiedlichen Erregern stammen können, oder es können Mischungen von Ghosts mit jeweils verschiedenen Antigen-kodierenden Nukleinsäuren verwendet werden.

In einer Ausführungsform der Erfindung können sogenannte homologe Kombinationen von Bakterienghost und Antigen-kodierender Nukleinsäure verwendet werden, wobei z.B. der Bakterienghost Oberflächenstrukturen trägt,die aus der gleichen Spezies oder dem gleichen Organismus stammen wie das von der Nukleinsäure-Vakzine kodierte Antigen. Gegebenenfalls kann der Ghost auf seiner Oberfläche sogar eine dem kodierten Antigen entsprechende Oberflächenstruktur tragen. Diese homologe Ghost/Nukleinsäure-Kombination kommt insbesondere zur Vakzinierung gegen bakterielle Infektionen in Frage, sie kann jedoch - bei Verwendung von rekombinanten Ghosts mit entsprechenden Oberflächenstrukturen - auch auf die Vakzinierung gegen andere Erkrankungen, z.B. virale Erkrankungen, erweitert werden.

Alternativ dazu wird eine heterologe Ghost/Nukleinsäure-Kombination verwendet. Bei einer derartigen heterologen Kombination erfüllt der Bakterienghost im allgemeinen Adjuvansfunktionen. Es sind jedoch auch Ausführungsformen möglich, bei denen ein aus einem pathogenen Bakterium stammender Ghost in Kombination mit einer heterologen Nukleinsäure als Kombinationsvakzine gegen zwei unterschiedliche Erreger verwendet wird.

Schließlich sind die Bakterienghosts auch als Träger- oder Targetingvehikel für den Agrarbereich geeignet, wo sie zum Ausbringen von Wirkstoffen wie etwa Herbiziden, Fungiziden oder/und Insektiziden verwendet werden können.

Die pharmazeutische Verabreichung der Wirkstoff-enthaltenden Ghosts kann nach geläufigen Methoden erfolgen, beispielsweise oral, aerogen, z.B. intranasal, intraokulär, topisch oder parenteral, z.B. intramuskulär, intraperitonial, intravenös oder subkutan.

Die Verabreichung der Ghosts erfolgt vorzugsweise auf demselben Weg, wie auch eine natürliche Infektion des Organismus mit dem Erreger erfolgt. So können Bakterienghosts mit Wirkstoffen, die zur Bekämpfung von pathogenen Erregern vorgesehen sind, deren Hautpeintrittspforte der Gastro-intestinaltrakt ist (E.coli, Salmonella, Vibrio oder Helicobacter), oral verabreicht werden. Ghosts aus Erregern von Lungenentzündungen, die entsprechende Wirkstoffe enthalten, z.B. Actinobacillus, Pasteurella, Pseudomonas oder Haemophilus, werden vorzugsweise aerogen verabreicht.

Die erfindungsgemäße Verabreichung von Bakterienghosts mit Wirkstoffen ist nicht nur für die Humanmedizin, sondern auch für die Tiermedizin, insbesondere für die protektive Impfung von Haustieren und Nutztieren, wie etwa Hunden, Katzen, Schweinen, Kühen etc. geeignet.

Für landwirtschaftliche Anwendungsformen können die Ghosts über den Boden, die Luft und Wasser oder als Kapseln an Samen verabreicht werden.

Die Applikation von Wirkstoffen über Bakterienghosts hat gegenüber bisherigen Applikationsformen eine Vielzahl von Vorteilen. So reichen bereits geringe Wirkstoffmengen zur Erzielung einer starken Wirkung aus. Weiterhin ist eine Zielzellen/Gewebe-spezifische Verabreichung der Wirkstoffe möglich. Aufgrund der bereits für sich immunogen wirkenden Bakterienghosthüllen wird eine Adjuvanswirkung erzielt. Der in den Ghost eingeschlossene Wirkstoff ist vor Abbau durch physiologische Prozesse, z.B. durch Enzyme wie Proteasen, Nukleasen oder Hydrolasen geschützt. Darüber hinaus ist eine Kombination mit weiteren Wirkstoffen möglich. Schließlich können die Bakterienghosts kostengünstig hergestellt und die Wirkstoffe einfach und kostengünstig formuliert werden.

Verschlossene Bakterienghosts mit metabolischen Funktionen bzw. mit der Fähigkeit zur Vermehrung, die im Vergleich zu einer natürlichen Zelle ein funktionell limitiertes Genom enthalten, können für die Erforschung zellulärer Prozesse eingesetzt werden. Weiterhin können die rekonstituierten Ghosts beispielsweise als attenuierte Lebendvakzine eingesetzt werden, da sich der Attenuierungsgrad aufgrund entsprechender Manipulationen im Genom sehr einfach und zuverlässig steuern lässt. Schließlich können die funktionell rekonstituierten Ghosts auch in der Biotechnologie, z.B. als "Reaktoren" zur Produktion rekombinanter Proteine, insbesondere rekombinanter humaner Proteine in großtechnischen Prozessen eingesetzt werden. Die erfindungsgemäßen Zellen mit retardiertem Genom weisen eine im Vergleich zur Ausgangszelle eine erheblich geringere Komplexität des Stoffwechsels auf und können daher gezielt manipuliert werden, um höhere Produktionsleistungen zu erreichen. Ein weiterer Vorteil ist die höhere Sicherheit, da die Zelle im Vergleich zu einer natürlichen Bakterienzelle ein erheblich geringeres Überlebenspotential besitzen.

Weiterhin soll die Erfindung durch die nachfolgenden Figuren und Beispiele erläutert werden. Es zeigen:

| | |
|---|---|
| Abb. 1: | Schema zur Wirkung von Cobalt und Kupferionen |
| Abb. 2: | Ghosts befüllt mit fluoreszierender DNA - inkl. entsprechender Fotots nach Hoechst 33324-Färbung |
| Abb. 3: | Ghosts befüllt mit fluoreszierender DNA - mit und ohne Cobalt- bzw. mit Kupferzugabe |
| Abb. 4: | Ghosts befüllt mit und ohne Calcein - mit und ohne Vesikel bzw. mit und ohne Calcium |

### Beispiel 1

### 1. Material und Methoden

### 1.1. Herstellung von E.coli Ghosts

E.coli NM522-Zellen (Stratagene) wurden mit dem Lyseplasmid pML1 (Szostak et al., J. Biotechnol. 44 (1996), 161-170) transformiert. Die Transformanten wurden in LB-Medium (10 g/l Trypton, 5 g/l Hefeextrakt, 5 g/l NaCl) mit Antibiotikum bei 28°C kultiviert. 1 I Medium wurde mit einer Übernachtkultur inokuliert, die aus einer einzelnen Transformantenkolonie stammte, und als Vorkultur für einen Fermenter (Typ MRD 60TE, Meredos GmbH, Bovenden, Deutschland) verwendet. Die Bakterien wurden im Fermenter in einem Volumen von 10 1 unter Belüftung und Rühren bis zum Erreichen einer optischen Dichte bei 600 nm von 0,4 kultiviert. Nach 30 min wurden 0,2 M MgSO₄ zugegeben und 20 min danach wurde die Expression des Lyseproteins E durch Temperaturerhöhung von 28°C auf 42°C induziert. Nach 1 h wurden die Zellen durch Zentrifugation bei 4000 g geerntet. Die Resuspension der Pellets in destilliertem Wasser (Endvolumen 5 I) führte zu einer sofortigen Lyse. Die Ghosts wurden zweimal in einem großen Volumen von Trisgepufferter Salzlösung (TBS) gewaschen und anschließend lyophilisiert.

### 1.2 Herstellung von Membran-Lipidvesikein

E.coli NM522 Zellen wurden in LB-Medium bei 37°C kultiviert und in der späten logarithmischen Wachstumsphase geerntet. Nach dreimaligem Waschen mit Phostphat-gepufferter Salzlösung (PBS) pH 7,4 wurden die Zellen in einem Aliquot PBS resuspendiert (Proteinkonzentration 26 mg/ml) und bei -70°C eingefroren. 10 ml dieser Suspension wurden nach dem Auftauen in einer Frenchpress bei 900 psi (große Zelle) gepresst. Zellreste und größere Fragmente wurden durch Zentrifugation (6000 UpM 10 min) entfernt. Die im Überstand enthaltenen Vesikel wurden durch Ultrazentrifugation bei 285.000 g (60 min) pelletiert und danach im Trispuffer pH 7,5 aufgenommen (Proteinkonzentration ca. 4 mg/ml). Die Vesikelsuspension kann bei 4°C ca. 1 Woche gelagert werden.

### 1.3 Herstellung von fusionskompetenten Bakterienghosts

Die Zellen einer Ghostsuspension (Proteinkonzentration 4 bis 6 mg/ml) wurden durch Zentrifugation pelletiert und einmal mit dem gleichen Volumen eiskalten 100 mM CaCl₂ gewaschen. Vor der weiteren Verwendung wurden die Ghosts 2 bis 3 h auf Eis gelagert bzw. bei -70°C eingefroren und vor Gebrauch auf Eis aufgetaut. Dieser Schritt ist für das Verschließen der Ghosts jedoch nicht zwingend notwendig.

### 1.4 Befüllen von Bakterienghosts mit Wirkstoffen

75 µl einer Ghostsuspension wurden bei 13.000 UpM (5 min) pelletiert und in 75 µl Fusionspuffer (100 mM NaCl, 10 mM Natriumacetat, 10 mM Hepes pH 5, 6 oder 7) aufgenommen, wobei der zu befüllende Wirkstoff bereits im Puffer gelöst war. Beispiele für Wirkstoffe waren ONPG (8 mg/ml), Calcein (2'2'-Bis[N,N-bis(carboxymethyl)-aminomethyl]-fluorescein; MW622,5; Fluka Austria; 0,66 mM), 5'-Fluorescin markierte DNA (Länge: 400 bp) und Sulforhodamin (MW 580,6; Sigma; 10 mM).

Die Herstellung der fluoreszenzmarkierten DNA war wie folgt:
400 bp der DNA des archaebakteriellen Phagen θCH1 wurden mittels Polymerasekettenreaktion (PCR) amplifiziert und gleichzeitig durch die Fluorescein-markierten Primer (Oligo sequencing service, Universität Wien) am 5'-Ende markiert. Die Reaktion (1,75 nM dNTPs, 0,5 µM der Primer, 1 ng/µl Phagen DNA, 0,02 U/µl Taq DNA Polymerase, Puffer) erfolgte unter folgenden Bedingungen: 4 min Prädenaturation, 35 Zyklen: 30 sec 94 °C/ 30 sec 60 °C/2 min 68 °. Die Aufreinigung des DNA-Fragments wurde mit Qiagen Purification Kit durchgeführt.

### 1.5 Fusion von Bakterienghosts mit Membran-Lipidvesikeln

Zu 75 µl Fusionspuffer gemäß 1.4 wurden nach einer ein- bis zweistündigen Inkubation bei 28°C 75 µl Vesikelsuspension und Calcium (Endkonzentration 25 mM) gegeben. Nach kurzem Vortexen wurde die Suspension bei 37°C über Nacht inkubiert. Anschließend erfolgte eine Ultrazentrifugation (50.000 UpM im TLA 100.3 Rotor) für 30 min bei 37°C.

### 1.6 Nachweis der Verpackung von Wirkstoffen

### 1.6.1 OPNG

Die durch Ultrazentrifugation pelletierten verschlossenen Ghosts wurden nun mindestens dreimal mit Tris-Puffer pH 7,5 gewaschen bis kein ONPG mehr im Überstand festgestellt werden konnte. Anschließend wurden die verschlossenen Ghosts mit Chloroform/SDS (je 33 µl) versetzt, gevortext, 1 min bei 13.000 UpM zentrifugiert, und der Überstand mit dem Zellinhalt abgehoben. Auf diese Weise wurde das verpackte ONPG freigesetzt, und konnte anschließend nach Zugabe von β-Galactosidase und Umsetzung zu einem gelben Farbstoff nachgewiesen werden. Dieser Befund zeigt, dass ein Verschluß der Ghosts und eine Verkapselung des Wirkstoffs stattgefunden hat.

Als Kontrollen wurden Fusionsansätze durchgeführt, die keine Vesikel, keine ONPG oder keine Bakterienghosts enthalten. So wurde sichergestellt, daß ONPG nicht unspezifisch an Ghosts bzw. Vesikel bindet, bzw. ONPG nicht im Vesikel eingeschlossen wird.

### 1.6.2 Calcein und DNA

Ghosts, welche mit Calcein bzw. markierter DNA befüllt worden waren, wurden im Fluoreszenzmikroskop bzw. mittels FACS (FACS Calibur, Becton Dickinson) nach Zugabe von Co²⁺ (50 mM) oder Cu²⁺ (1 % w/v) untersucht. Da nach Zugabe von Cobalt, welches Membranen nicht penetriert, aber die Fluoreszenz von Calcein bzw. Fluorescin-markierter DNA effektiv quencht, immer noch fluoreszierende Ghosts nachgewiesen werden konnten, nach Zugabe des Membran-penetrierenden Kupfers (welches auch ein sehr wirksames Agens zum Quenchen der Fluoreszenz von Calcein und Fluorescin-markierter DNA ist), aber keine Fluoreszenz beobachtet werden konnte, ist dies ein eindeutiger Hinweis für die erfolgreiche Membranfusion, also die Wiederherstellung der Integrität der Ghostmembran.

Durch zusätzliches Anfärben des verpackten 400 bp-Fragments (sowie der Ghost-DNA) mit dem Membran-penetrierenden DNA-Farbstoff Hoechst 33342, welcher nur in intakte DNA interkaliert, Nukleotide aber nicht anfärbt, und Anregung des interkalierten Farbstoffes mit UV konnte mittels DAPI-Filter Fluoreszenz beobachtet und somit bewiesen werden, dass das 400 bp-Fragment noch intakt in den geschlossenen Ghosts vorliegt.

### 2. Ergebnisse

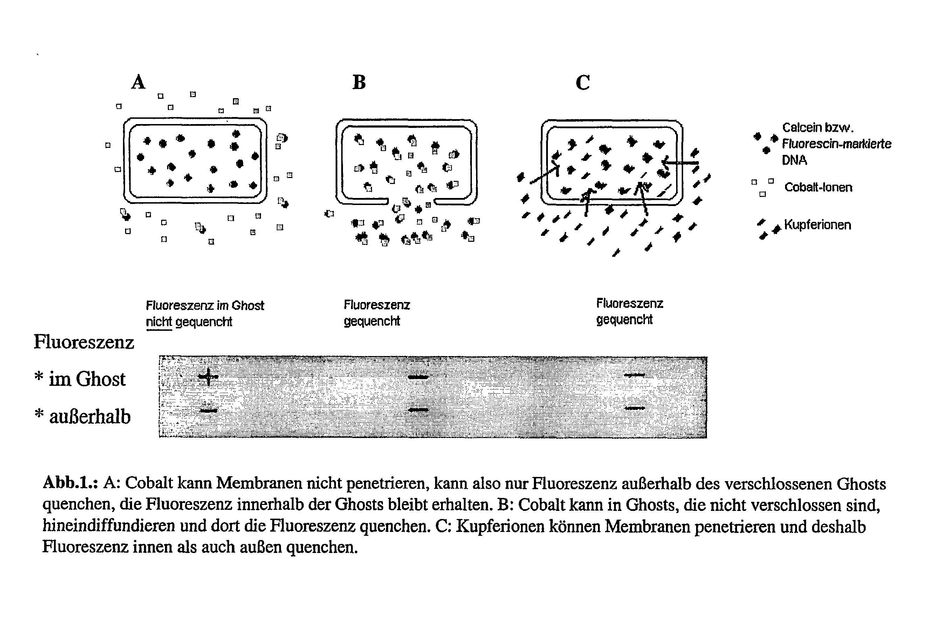

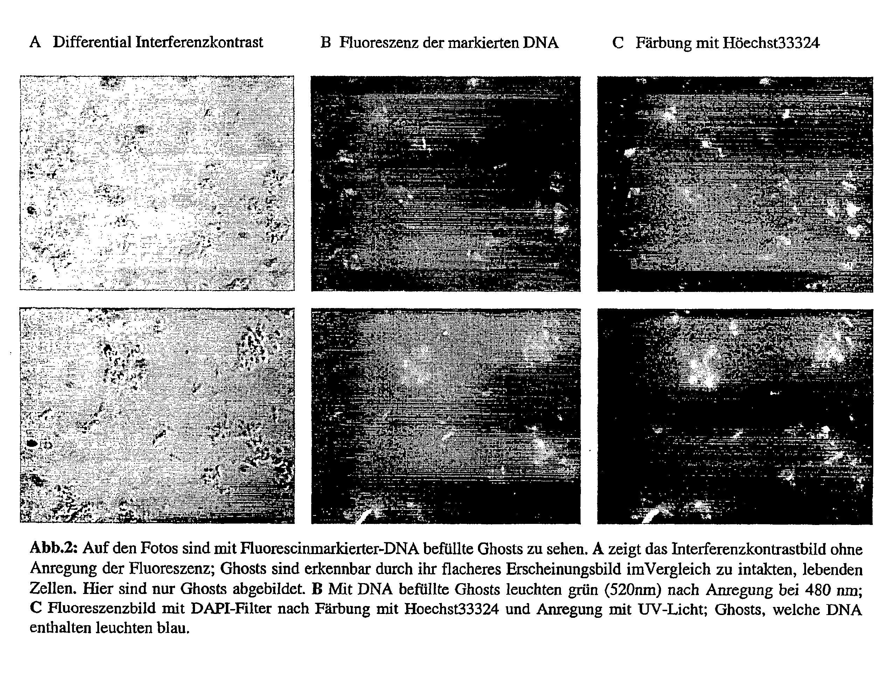

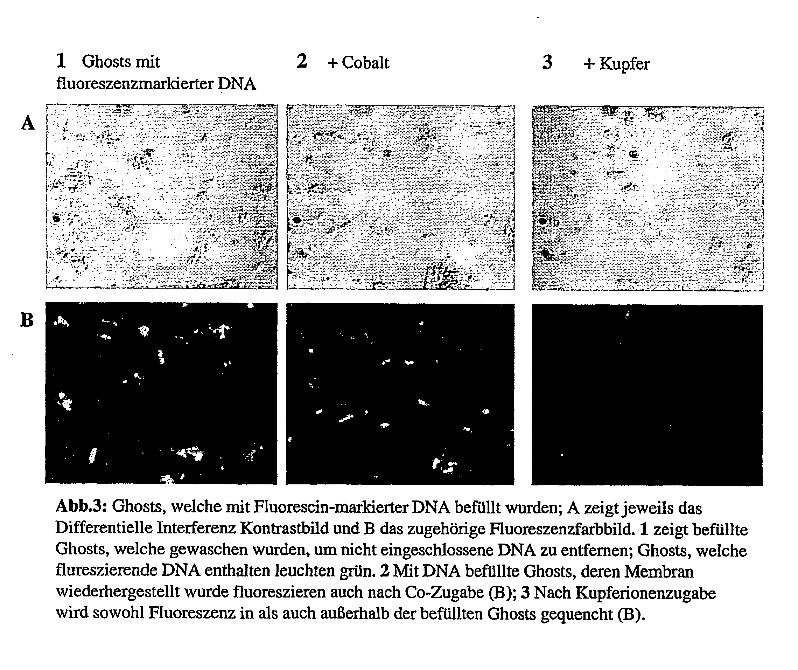

Mehrere verschiedene Substanzen, welche verschiedene Eigenschaften aufweisen, wurden für die Befüllung der Ghosts verwendet. ONPG als ein wasserlösliches Substrat mit einem niedrigen Molekulargewicht für β-Galactosidase, welches nach Enzymreaktion photometrisch nachgewiesen werden kann. Fluorescein-markierte DNA, ein vergleichsweise großes Molekül, und Calcein, ein ebenfalls hydrophiles, kleines Molekül, dienten zur optischen Überprüfung der befüllten Ghosts durch Fluoreszenzmikroskopie. Die Fluoreszenz von Calcein (ein Derivat des Fluorescein) und Fluorescein (5'-Markierung der 400 bp-DNA) kann effektiv durch die Kationen Kupfer und Cobalt (D.A. Kendall, R.C. MacDonald, J. Biological Chemistry 257 (1982), 13892-13895); Oku N. et al., Biochim. Biophys. Acta 691 (1982), 332-340) gequencht werden, wobei Cu²⁺-Ionen Membranen penetrieren, Co²⁺-Ionen aber nicht. Der Zusatz von Cobalt hat zwei Effekte, nämlich erstens das Quenchen der Fluoreszenz des nicht eingeschlossenen, externen Calceins bzw. DNA und zweitens die Überprüfung der völligen Wiederherstellung der inneren Membran, durch welche Cobalt nicht diffundieren kann. Der Zusatz von Kupfer bewirkt ein völliges Quenchen der Fluoreszenz, da die Fluoreszenz sowohl des externen als auch des eingeschlossenen Calceins bzw. Fluoresceins (DNA-Markierung), siehe Abbildungen 1 bis 4.

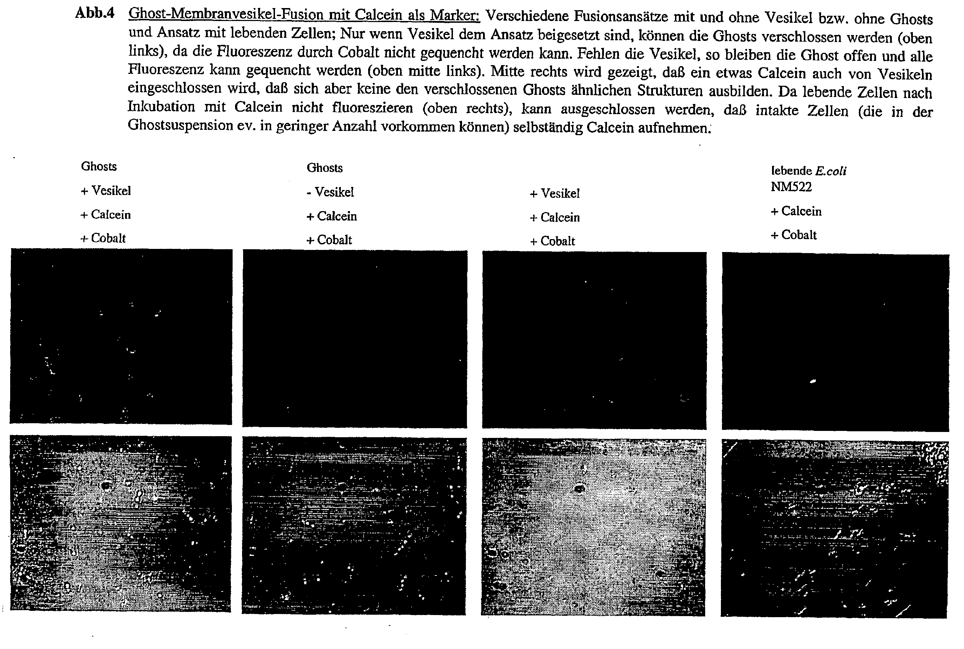

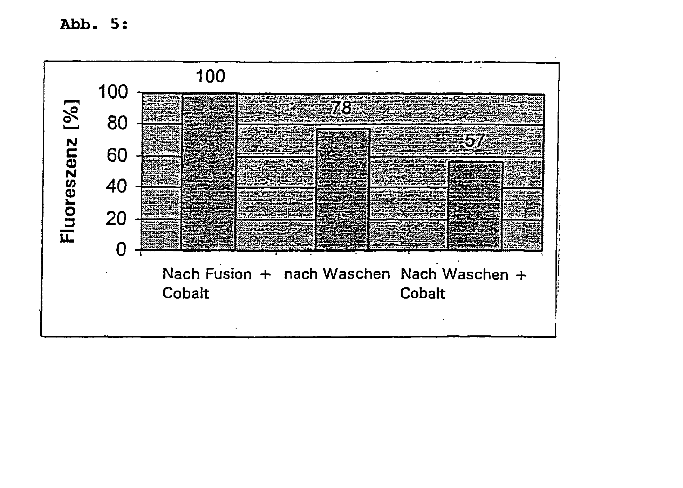

Ghosts, die mit Calcein (10mM) befüllt wurden, wurden nach Zugabe von Cobalt (50mM) im Mikroskop betrachtet. Die Quenchlösung hat den Vorteil, dass die Ghosts nicht gewaschen werden müssen und so auch nicht durch mechanische Einwirkung wieder geöffnet werden. Dies sollte ein ursprünglicheres Bild liefern.
Außerdem wurden befülllte Ghosts gewaschen und dann mit und ohne Cobalt fotografiert und ausgezählt (siehe Abb. 4). In dieser Abbildung werden befüllte und verschlossene Ghosts, beladene Ghosts ohne Vesikel, Vesikel, welche durch die Befüllungsreaktion geschleust wurden und intakte Zellen des Stammes E.coli NM522, welche mit Calcein inkubiert worden waren, nach Cobaltzugabe gezeigt. Es ist ersichtlich, daß Vesikel zum Verschluss der Ghosts bzw. zur völligen Wiederherstellung der Ghostmembran nötig sind, da die überwiegende Mehrzahl aller beladenen Ghosts nicht fluoreszieren. Vesikel nehmen selbst auch Calcein während der Befüllungsreaktion auf, welches dann für Cobaltionen nicht zugänglich ist. Außerdem konnte gezeigt werden, dass intakte Bakterien Calcein nicht unspezifisch binden oder aktiv aufnehmen.
Aufgrund der mikroskopischen Fotos, welche vor und nach Waschen in Gegenwart von Cobalt gemacht wurden ergeben sich folgende Daten. (Die Zugabe von Quencher ist im Fall des Calceins obligat, da aufgrund der hohen Fluoreszenz des freien Calceins keine Fotos gemacht werden können.) Abhängig von der Qualität und vom Alter der Vesikel emittieren 20-60% (im Durchschnitt 38%) aller Ghosts grüne Fluoreszenz nach Cobalt-Zugabe. Der Graph in Abb. 5 zeigt den Einfluß des Waschens auf die Ghosts. Die Anzahl der fluoresziernden Ghosts verringerte sich nach Waschen um 22 %, nach zusätzlicher Cobalt-Zugabe um weitere 21 %, also insgesamt um 43 %. Das heißt, dass 57% der ursprünglich verschlossenen Ghosts auch nach einmaligem Waschen verschlossen bleiben bzw. noch immer genug Calcein in den Ghosts verbleibt, um ein Fluoreszenzsignal zu liefern.

Abb. 5: Einfluss des Waschens auf die Ghosts; Angegeben werden die verschlossenen Ghosts nach Cobalt-Zugabe. Als 100% wurden die ursprünglich, d.h. vor dem Waschen, verschlossenen Ghosts gewählt.

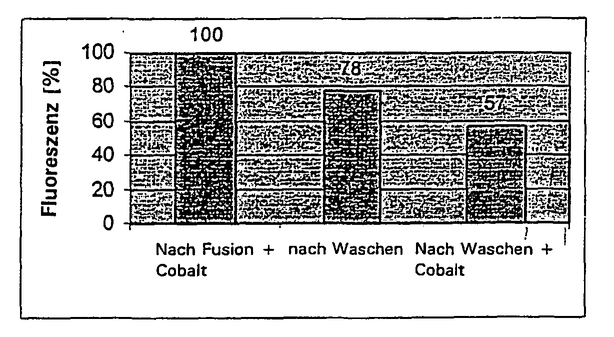

Da Cobaltionen die Fluoreszenz des Calceins nicht zu 100% quenchen kann, bleibt in jedem Fall eine gewisse Restfluoreszenz zurück, die vom Auge ev. auch noch hell empfunden bzw. fotografiert werden kann. Deshalb ist eine quantitative Bestimmung der Fluoreszenz für jeden einzelnen Ghost wichtig. Nur so kann eine exakte Determination der Rate der beladenen und beladenen und verschlossenen Ghosts erfolgen. Hierfür wurde die Durchflußcytometrie (FACS) eingesetzt.
Wenn keine Quenchlösung zugegeben wird bzw. fluoreszenzmarkierte DNA oder SulforhodaminB für die Beladung verwendet werden, dann sieht man (Mikroskop), dass > 90% der Ghosts mit den Reportersubstanzen beladen werden können. Die Beladungseffizienz ist also sehr hoch, wie bereits durch den Versuch mit ONPG gezeigt werden konnte. Über die Verschlussrate kann so keine Aussage getroffen werden.

Ghosts, welche mit fluoreszierender DNA (DNA*) bzw. mit Calcein befüllt wurden, werden getrennt diskutiert, da sich Unterschiede in der Quenchbarkeit der beiden Reportersubstanzen ergeben haben. Es wurden folgende Versuchsansätze nach dem bestehenden Verschlussprotokoll durchgeführt:
1. ECG + Calcein oder DNA* + Vesikel
2. ECG + Calcein oder DNA* - Vesikel
3. ECG + Calcein oder DNA* + Vesikel
4. Calcein oder DNA* + Vesikel
5. Vesikel

Wurde eine Komponente im Versuchsansatz weggelassen, so wurde diese durch eine äquivalente Menge Puffer ersetzt, so dass sich immer die gleichen Calcein-bzw, DNA*-Konzentrationen ergeben.

Beladene und verschlossene Ghosts wurden definiert als die Partikel im Ghostgate, welche nach Zusatz von Cobalt eine Fluoreszenz von > 8% der Fluoreszenz vor Cobaltzugabe aufweisen. (Begründung: Cobalt quencht mind. 92% aber maximal 94% der Fluoreszenz des Calceins.) Hier eine Zusammenfassung der an verschiedenen Versuchstagen gemessenen Verschlußraten (siehe Tab. 1).

**Tab. 1: Prozentsatz der verschlossenen Ghosts (ECG) in einer Ghostssuspension; die beiden parallel geführten Versuchsansätze (a-e) unterscheiden sich lediglich durch An- bzw. Abwesenheit der Membranvesikel.**

| ECG+Calcein+Vesikel | | ECG+Calcein-Vesikel |
|---|---|---|
| ^{Versuchsansatz} % verschlossene Ghosts | | % verschlossene Ghosts |
| a | 3,27 | 4,15 |
| b | 43,34 | 33,34 |
| c | 11,02 | 12,17 |
| d | 0,17 | 0,62 |
| e | 0,62 | 0 |

Das heißt, dass 0,62 bis 43,45 % aller dedektierten Ghosts mit Calcein beladen und verschlossen worden sind.
Werte aus den Versuchsansätzen 3, 4 und 5 dienten als Blindwerte, um welche die angegebenen Verschlusseffizienzen bereits korrigiert worden waren. Es ergeben sich hier wie bei der mikroskopischen Messung sehr große Schwankungen in den Verschlussraten. Die Verschlussraten in den Versuchsansätzen d und e (0 + 0,62%) sind als negativ zu werten, Verschlusseffizienzen < 3% also Background sind. Schwankungen der Verschlusseffizienzen können durch unterschiedliche Membranvesikelpräparationen oder durch die unterschiedlich lange Lagerung der Ghosts erklärt werden. Möglicherweise hat die Dauer und Art der Lagerung Einfluss auf die Verschlussfähigkeit der Ghosts. (Lagerbedingungen: Ghosts in PBS, -80° C)

Aus den gezeigten FACS-Daten ist auch ersichtlich, dass Vesikel für den Verschluss nicht zwingend notwendig sind. Man kann davon ausgehen, dass in der Ghostspräparation noch sehr viele Membranvesikel enthalten sind und dass unter Umständen die Versuchsbedingungen die Fusion von Ghosts untereinander begünstigen. Die Daten in Tabelle 1 zeigen aber auch, dass ein vollständiges Quenchen der Fluoreszenz des Calceins in den nicht verschlossenen Ghosts möglich ist.

### Fluorescin-markierte DNA

Nach meinen fluorimetrischen Messungen der relativ schlechten Quenbarkeit der freien Fluorescin-markierten DNA durch Cobalt und Kupfer ergibt sich folgende Definition für beladene und verschlossene Ghosts: Die Partikel, deren Fluoreszenz nach Cobaltaddition > 40% der Fluoreszenz vor Cobaltzugabe beträgt, wurden als verschlossen gewertet. Hier eine Zusammenfassung der an verschiedenen Versuchstagen gemessenen Verschlussraten (siehe Tab. 2).

**Tab. 2: Prozentsatz der verschlossenen Ghosts nach Cobalt-Zugabe; Daten wurden durch Durchflußcytometrie (FACS Calibur) ermittelt.**

| mit Ultrazentrifuge | mit Ultrazentrifuge | ohne Ultrazentrifuge | ohne Ultrazentrifuge |
|---|---|---|---|
| ECG+DNA*+Vesikel | ECG+DNA*-Vesikel | ECG+DNA*-Vesikel | ECG+DNA*-Vesikel |
| % verschlossene Ghosts | % verschlossene Ghosts | % verschlossene Ghosts | % verschlossene Ghosts |
| 35,05 | 23.2 | 56,2 | 35,25 |
| 44 | 46,49 | 51,8 | 48,0 |
| 29,92 | 34,26 | 28,79 | |

Das heißt, dass durchschnittlich 36,3 % aller Ghosts mit DNA* beladen und verschlossen worden sind. Schwankungen in den Verschlussraten lassen sich wie im Beispiel des Calceins durch unterschiedliche Vesikelpräparationen und dem möglichen Alterungsprozess der Ghosts erklären. Außerdem ist hier ersichtlich, daß eine Ultrazentrifugation der Ghosts nicht unbedingt nötig ist, wenn eine absolute Abtrennung der Ghosts vom Überstand nicht zwingend nötig ist, wie z.B. in analytisch quantitativen Experimenten, in welchen die Beladungseffizienz gemessen wird.

### Beispiel 2

### Verpackung eines Translations- Transkriptionssystems und eines β-Galexpressionsplasmids

### 1. Material und Methoden

Die Aufreinigung des Repressors cl857 erfolgte nach den früher beschriebenen Methoden (Frinha MA, Keopinski AM; Can. J. Microbiol (1997) 43:220-226; Johnson AD, Pabo CO, Sauer RT; Methods Enzymol. (1980) 65:839-56). Für die Expression der β-Galactosidase wurde die Plasmide pCS-Iac (Jechlinger W, Szostak MP, Lubitz W; (1998) Gene 218: 1-7) und pAWJ-Iac (Jechlinger W, Szostak MP, Witte A, Lubitz W; (1999) FEMS Microbiol Letters 173: 347-352)verwendet. In beiden Plasmiden stehen die β-Galactosidasegene unter einem Temperatursensitiven Promoter. Beim pCS-lac erfolgt die Induktion der Enzymexpression durch Temperaturdownshift von 37° auf 28°C, beim pAWJ-lac durch Temperaturupshift von 37° auf 42°C.
Die Verpackung des Plasmides mit dem Transkriptions-Translations-Assay (Promega) erfolgte durch das bestehende Verpackungsprotokoll mit und ohne Membranvesikel. Der Transkriptions-Translations-Assay bestehend aus 2,5µl 10 mM Aminosäuremix, 10 µl S30-Premix, 7,5 µl S30-Extrakt und 2,5 µl 10 mM SMethionin wurde mit dem Plasmid (Konz. Maxipräparation, Qiagen) und dem aufgereinigten Repressor vermischt, die E.coli NM522-Ghosts damit bei 37°C durch Diffusion beladen und bei 37°C mit den Membranvesikeln verschlossen. Nach erfolgter Verschlussreaktion wurden die Ghosts 3 mal mit Fusionspuffer gewaschen und dann die Expression der β-Galactosidase durch Temperatur-down shift bzw. up shift (siehe voriger Absatz) induziert. Nach 0, 10, 20, 30 und 60 min im Fall des pAWJ-lac und nach 0, 1, 2, 3, 4 Stunden im Fall des pCS-lac wurde je ein Aliquot der Ghostsuspension entnommen, die Ghosts durch Glutaraldehyd fixiert und durch Zusatz von X-Gal (5-brom0-4-chloro-3-indolyi-β-D-galactosid; Färbelösung: 0,2%X-Gal, 2 mM MgCl₂, 5 mM K₄Fe(CN)₆.3H₂O, 5 mM K₃Fe(CN)₆ in PBS (phosphate buffered saline)) auf β-Galactosidaseexpression untersucht. Wird β-Galactosidase gebildet, so wird das ursprünglich farblose X-Gal in Lactose und das blaue Indol gespalten. Das heißt, dass β-Galactosidase exprimierende Zellen blau erscheinen, welche dann im Mikroskop bei einer 100fachen Vergrößerung gezählt wurden.

### 2. Ergebnis und Diskussion

Ghosts des Stammes *E.coli* NM522 wurden mit zwei β-Galactosidaseexpressionsplasmiden (pAWJ-lac und pCS-Iac) für Prokaryonte, dem Repressor c1857 und einem kommerziell erhältlichen Transkriptions-Translations-Assay beladen und mit Membranvesikeln verschlossen. Die Expression der β-Galactoxsidase wurde in den gewaschenen Ghosts durch Temperatur-up bzw. down shift induziert. Durch Zugabe von X-Gal konnte gezeigt werden, dass im Fall des pCS-Iac nach 3 h Inkubation bei 28°C 0,3 + 0,8% aller Ghosts leicht blau aber nach 4 h tief blau gefärbt waren, d.h. dass sie β-Galactosidase exprimierten und dass im Fall des pAWJ-lac bereits nach 20 min in 0,5-2% aller Ghosts eine β-Galexpression stattfindet. Erfolgte kein Verschluss der Ghosts, so wurde der Tk-Tl-Assay aus den Ghosts herausgewaschen und keine β-Galactosidase exprimiert, erfolgte also keine Blaufärbung. Erfolgte keine Induktion, so konnte ebenfalls keine β-Galactosidaseaktivität nachgewiesen werden.

### Beispiel 3

### Verpackung von pEGFP und Expression von GFP in verschiedenen Zelllinien

Durch eine Reihe von Versuchen wurde eine erfolgreiche Aufnahme von markierten Ghosts durch Colonendothelzellen, Makrophagen und dendritische Zellen und ein "drug release" gezeigt worden ist, wurde auch untersucht, ob potentiell ein gene targeting von eukaryontischen Zellen mit Ghosts möglich ist. Für diesen Versuch wurden Makrophagen RAW164.7 und Humane Colon Endothelzellen Caco-2 verwendet.
Das Plasmid pEGFP (Clontech), das für EGFP (enhanced green fluorescent protein) unter einem eukaryontischen Promotor (CMV) mit einem humanen Codonusage codiert, wurde in Ghosts verpackt, welche mit den Zellen inkubiert wurden. Sollte ein gene targeting möglich sein, so müßten die eukaryontischen Zellen EGFP exprimieren und so nach Anregung bei 488 nm grün fluoreszieren (Emmission max. 507 nm).

### 1. Material und Methoden

### Verwendete Stämme für die Ghostproduktion: E.coli NM522 und Vibrio cholerae

Die Verpackung des Plasmides pEGFP (Clontech,) erfolgte nach dem bestehenden Beladungsprotokoll mit und ohne Vesikeln, da im Falle der DNA nicht unbedingt ein Verschluss stattfinden muss, da wie bereits gezeigt die DNA an Ghosts bindet. Überschüssige DNA wurde durch Waschen der Ghosts und anschließendem DNasel-Verdau entfernt. Das verpackte Plasmid konnte durch PCR des codierten EGFP nachgewiesen werden.
Die eukaryontischen Zellen wurden vor Zugabe der Ghosts 3 -5 h in den entsprechenden Zellkulturschalen kultiviert. Die mit pEGFP beladenen Ghosts wurden auf die Zellen (Makrophagen RAW264.7; ATTC: HTP-37, Humane Colon Endothelzellen Caco-2; ATTC: TIB71) aufgebracht (MOI:10-100) und über Nacht inkubiert. Die nicht aufgenommenen Ghosts wurden dann mit dem Medium abgehoben, die Zellen mit PBS gewaschen und mit neuem Kulturmedium versehen. Nach einer weiteren Inkubation (insgesamt 48 h nach Zugabe der Ghosts) wurden die Zellen mit einem Fluoreszenzmikroskop auf GFP-Expression untersucht.
Beschreibung des pEGFP-N1 (Clontech, Genebank Accession U55762): human cytomegalovireus (CMV) immediate early promoter; enhanced green fluorescent protein gene (EGFP); SV40 origin of replication.

### 2. Ergebnis und Diskussion

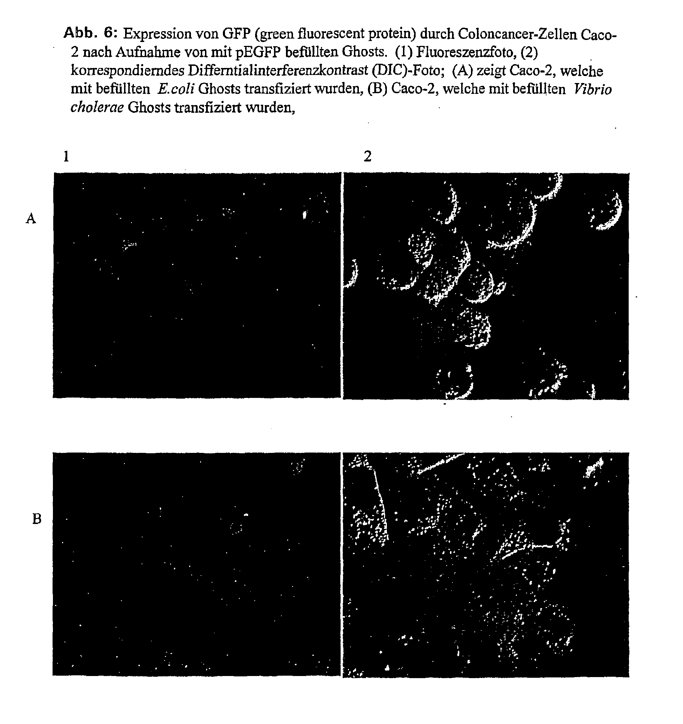

Im Inversmikroskop in den Zellkulturschalen wurde nach Transfektion mit *E.coli*-Ghosts im Fall der murinen Makrophagen RAW264.7 eine Expressionseffizienz des GFP von ca. 10⁻⁵ bis 10⁻⁴ beobachtet. Es wurden auch Fotos von Zellen, die in chamber slides kultiviert worden waren gemacht. Im Fall der humanen Coloncancerzellen Caco-2 exprimierten bei Verwendung von *E.coli*-Ghosts bis zu 10% und bei Verwendung von *V.cholerae*-Ghosts bis zu 7% der Zellen EGFP (siehe Abb. 6). Die Auswertung erfolgte durch Zählen am Fluoreszenzmikroskop.

### Beispiel 4

### Drug-release in Makrophagen, Caco-2 und HepG2

Um zu zeigen, dass wasserlösliche Wirkstoffe mit Ghosts transportiert und zu bestimmten Wirkorten gebracht und dort auch freigesetzt werden können, wurde Calcein in einer Konzentration, die ein Selbstquenching erlaubt, verpackt und auf Makrophagen, Caco-2 und HepG2, die Ghosts phagocytieren aufgebracht. Wenn Ghosts mit dem verpacktem Calcein nur phagocytiert werden und in Phagolysosomen "zerlegt" werden, so sollte man durch die Verdünnung des Calceins in den Kompartimenten Fluoreszenz beobachten können.

### 1. Material und Methoden

Ghosts wurden mit 200mM Calcein bzw. mit dem pH stabileren rot fluoreszierenden Farbstofft SulforhodaminB (10µM; Sigma) beladen und mit Vesikeln verschlossen wie bereits beschrieben wurde. Ab einer Konzentration von 200 mM bilden Calceinmoleküle mit sich selbst Komplexe, die nicht fluoreszieren, d.h. der Farbstoff quencht sich selbst. Die befüllten Ghosts wurden nach 24 h Dialyse im Fusionspuffer im Fluoreszenzmikroskop betrachtet und auf Selbstquenching kontrolliert. Als Kontrolle diente freies Calcein bzw. freies SulforhodaminB.
Makrophagen der Zelllinie RAW246.7 wurden mit den Ghosts 2 h inkubiert, dann mit PBS gewaschen und im Inversmikroskop sofort bzw. nach 24 h Inkubation nach Anregung bei 480 nm mit einem Emissionsfilter bei 520 nm betrachtet und fotografiert.

### 2. Ergebnisse und Diskussion

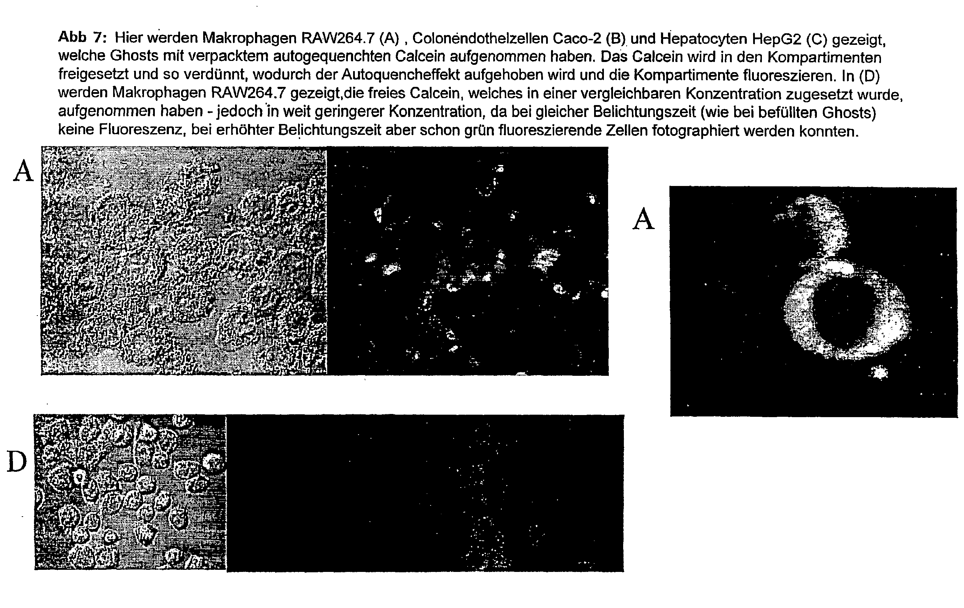

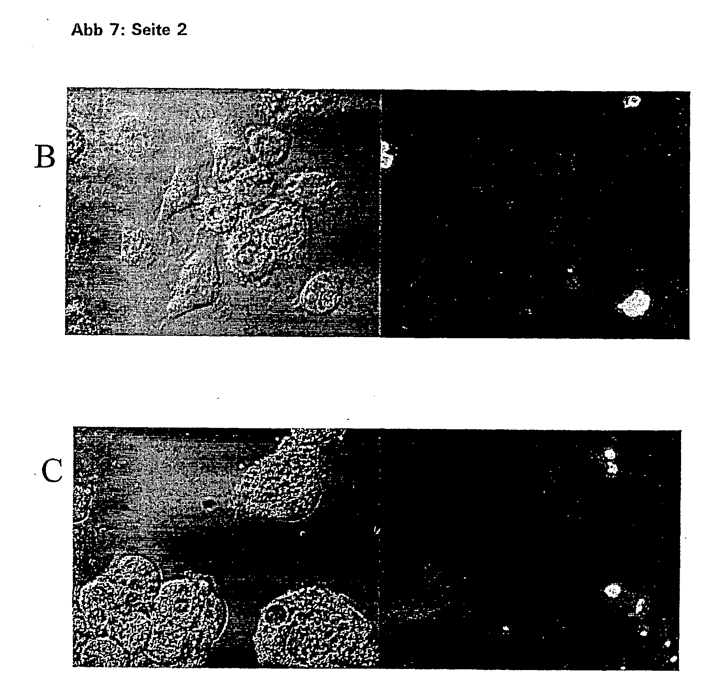

Ghosts, welche mit 200 mM Calcein befüllt worden waren (~30-40%), zeigten im Fluoreszenzmikroskop keine Fluoreszenz, was auf eine erfolgreiche Verpackung des selbstgequenchten Calceins schließen lässt. Hier konnte gezeigt werden, dass fast alle Makrophagen beladene Ghosts aufnehmen und das Calcein bzw. Sulforhodamin in vesikulären Kompartimenten, wie z.B. Endosomen, Lysosomen oder Endolysosomen) des Cytoplasmas freisetzen, jedoch nicht im Zellkern, welcher dunkel erscheint. Die Freisetzung des Calceins in den Kompartimenten bewirkte eine Verringerung der Konzentration und so das Aufheben des Autoquencheffektes, wodurch bewiesen ist, dass eine WirkstoffFreisetzung in Makrophagen grundsätzlich möglich ist. Freies Calcein wurde ebenfalls von Makrophagen aufgenommen, jedoch in wesentlich geringeren Konzentrationen. Gleiches konnte sowohl in humanen Coloncancer-Zellen (Caco-2) und humanen Hepatocyten (HepG2) wenn auch mit geringerer Effizienz gezeigt werden (siehe Abb. 7).

### Beispiel 5

### Verpackung von Druckerfarbe

Rote Druckerfarbe wurde in Ghosts verpackt und die Eigenschaften der verpackten Farbe im Vergleich zur Farbe allein in Hinblick auf Haftungseigenschaften auf Papier und Nitrozellulose untersucht.

### 1. Material und Methoden

Verwendet wurden Ghosts des Stammes *Escherichia coli* NM522. Verpackt wurde rote Druckerfarbe, welche 3 Minuten bei 13 000 UpM zentrifugiert wurde, um große Pigmente zu entfernen.
Nach Verpacken der Farbe wurden die Ghosts mehrmals mit Saline gewaschen, um nicht verpackte bzw. überschüssige Farbe zu entfernen.

Sowohl verpackte Farbe als auch nicht verpackte Farbe wurden nun auf Papier bzw. Nitrozellulosemembran aufgetragen. Nach Trocknen wurde das Papier bzw. die Membran verschiedenen Umweltfaktoren wie z.B. Hitze (60°C), Licht, Wasser, Ethanol oder Chloroform ausgesetzt. Beobachtet wurden die Intensität der Farbe bzw. der verpackten Farbe und die Haftungseigenschaften der Ghosts.
Der Nachweis der Ghosts erfolgte durch Anfärben der DNA, welche noch in den Ghosts vorhanden war, mit Ethidiumbromid. Die Membranen bzw. das Papier wurde für 1 Minute in Ethidiumbromid eingelegt, kurz mit Wasser gewaschen und dann unter einer UV-Lampe (265 nm) untersucht. Ethidiumbromid interkaliert in DNA und fluoresziert dann nach Anregung mit UV-Licht hellrot. Aufgrund der Tatsache, dass weißes Papier UV-Licht reflektiert, wurde rotes Papier verwendet, da auf diesem die hellrote Fluoreszenz des Ethidium-bromid-DNA Komplexes besser beobachtet werden konnte.

### 2. Ergebnis:

Die Beobachtungen bezüglich der Haftungseigenschaften der Ghosts bzw. der Farbe werden in nachstehender Tabelle wiedergegeben.
Der positive Nachweis der Farbe bzw. der verpackten Farbe wird durch ein + bzw. der negative Nachweis durch ein - wiedergegeben. (+) wurde verwendet, wenn die Farbintensität bzw. das Fluoreszenzsignal der Ghosts schwächer wurden.

| Behandlungsmethode | Papier | | Nitrozellulose | |
|---|---|---|---|---|
| | Farbe | Ghosts | Farbe | Ghosts |
| HITZE (60°C): 7 Tage bei 60°C im Trockenschrank | + | + | + | + |
| LICHT: 1 Monat bei einem südseitigen Fenster einer Lichteinstrahlung ausgesetzt | + | + | + | + |
| WASSER: | | | | |
| • 3 x mit Wasser je 5 min gewaschen • 7 Tage in Wasserbad bei Raumtemperatur eingelegt | + (+) | + (+) | + + | + + |
| ETHANOL: | | | | |
| • 3 x mit Ethanol je 5 min gewaschen • 7 Tage in Ethanol bei Raumtemperatur eingelegt | + (+) | + (+) | + + | + + |
| CHLOROFORM: | | | | |
| 1 x mit Chloroform gewaschen | (+) | + | (+) | + |

### 3. Zusammenfassung

Ghosts haften auf Nitrozellulose grundsätzlich besser als auf Papier. Hitze, Licht und mehrmaliges Waschen mit Wasser oder Ethanol haben kaum Einfluss auf die Haftung der Ghosts bzw. der Farbe. Nach Einlegen der Membran bzw. des Papiers in Wasser bzw. Ethanol für die Dauer von einer Woche konnte ein leichter Auswaschungseffekt sowohl der Farbe als auch der Ghosts beobachtet werden. Vergleicht man Ethanol und Wasser, so ist die Farbe durch Ethanol besser abwaschbar als die Ghosts, die Ghosts durch Wasser jedoch besser abwaschbar als die Farbe.
Grundsätzlich muss jedoch festgestellt werden, dass die Farbe nicht besser oder schlechter an Papier bzw. Nitrozellulose haftet, wenn diese in Ghosts verpackt bzw. mit Ghosts vermischt wurde.

Außerdem dürfte ein Vermischen dieser Farbe mit den Ghosts ausreichen, da die feinen Pigmente vermutlich an den Ghosts haften. Eine Verpackung der Farbe in den Ghosts wäre im Falle dieser Farbe also nicht unbedingt notwendig.
Der Vorteil der Ghost-Farbe Mischung liegt weniger in den verbesserten Haftungseigenschaften der Farbe als vielmehr in den antigenen Eigenschaften der Ghosts, da deren DNA bzw. deren Proteine an der Zelloberfläche in eindeutiger Weise durch molekularbiologische Methoden nachgewiesen werden kann und die Farbe so fälschungssicher wird.

## Patentansprüche

1. Verfahren zum Herstellen von verschlossenen Bakterienghosts umfassend das Inkontaktbringen von kompetenten Bakterienghosts mit Membran-Lipidvesikeln unter Bedingungen, bei denen die elektrostatische Abstoßung zwischen den Bakterienghosts und den Lipidvesikeln überwunden wird und bei denen die Membran in den Ausgangsmaterialien destabilisiert wird, so dass eine Fusion zwischen der Membran der Bakterienghosts und der Membran der Membran-Lipidvesikel erfolgt.

2. Verfahren nach Anspruch 1,
**dadurch gekennzeichnet,**
**dass** die Ghosts von Gram-negativen Bakterien stammen.

3. Verfahren nach Anspruch 1 oder 2,
**dadurch gekennzeichnet,**
**dass** die Ghosts von Bakterien stammen, ausgewählt aus der Gruppe bestehend aus Escherichia coli, Klebsiella, Salmonella, Enterobacter, Pseudomonas, Vibrio, Actinobacillus, Haemophilus, Pasteurella, Bordetella, Helicobacter, Francisella, Brambamella, Erwinia, Pantoea, Streptomyces, Frankia, Serratia, Agrobacterium, Azotobacter, Bradyrhizobium, Burkholderia, Rhizobium, Rhizomonas und Sphingomonas.

4. Verfahren nach einem der vorhergehenden Ansprüche,
**dadurch gekennzeichnet,**
**dass** die Ghosts von rekombinanten Bakterien mit heterologen Membranpolypeptiden stammen.

5. Verfahren nach einem der vorhergehenden Ansprüche,
**dadurch gekennzeichnet,**
**dass** die Bakterienghosts durch Inkontaktbringen mit mehrwertigen Metallkationen, insbesondere Calcium, und Inkubation bei einer Temperatur von 0 bis 5°C fusogen gemacht werden.

6. Verfahren nach einem der vorhergehenden Ansprüche,
**dadurch gekennzeichnet,**
**dass** als Lipidvesikel Vesikel von homogenisierten Zellen, insbesondere Bakterienzellen verwendet werden.

7. Verfahren nach einem der vorhergehenden Ansprüche,
**dadurch gekennzeichnet,**
**dass** als Lipidvesikel Liposomen verwendet werden.

8. Verfahren nach einem der vorhergehenden Ansprüche,
**dadurch gekennzeichnet,**
**dass** als Lipidvesikel membranumhüllte Viren verwendet werden.

9. Verfahren nach einem der vorhergehenden Ansprüche,
**dadurch gekennzeichnet,**
**dass** die Fusion zwischen der Membran der Bakterienghosts und der Membran der Lipidvesikel in Gegenwart von zweiwertigen Metallkationen, insbesondere Calciumionen oder/und organischen Aggregationsmitteln, insbesondere Glycerin, Polyethylenglykol oder Dimethylsulfoxid, erfolgt.

10. Verfahren nach einem der vorhergehenden Ansprüche,
**dadurch gekennzeichnet,**
**dass** die Fusion zwischen der Membran der Bakterienghosts und der Membran der Lipidvesikel unter Bedingungen erfolgt, bei denen die Membranen in einem flüssigen Zustand sind.

11. Verfahren nach einem der vorhergehenden Ansprüche,
**dadurch gekennzeichnet**,
weiterhin umfassend das Verpacken von Wirkstoffen in die Bakterienghosts.

12. Verfahren nach Anspruch 11,
**dadurch gekennzeichnet,**
**dass** die Wirkstoffe ausgewählt werden aus genetischem Material, Zellkomponenten, Substanzen, Markierungssubstanzen, landwirtschftlich wirksamen Substanzen, Farbstoffen und Kombinationen davon.

13. Verfahren nach Anspruch 11 oder 12,
**dadurch gekennzeichnet,**
**dass** die Wirkstoffe in den Bakterienghosts in immobilisierter Form vorliegen.

14. Verfahren nach Anspruch 11 oder 12,
**dadurch gekennzeichnet,**
**dass** die Wirkstoffe in den Bakterienghosts in freier Form vorliegen.

15. Verfahren nach Anspruch 12 oder 14,
**dadurch gekennzeichnet,**
**dass** genetisches Material und gegebenenfalls Zellkomponenten verpackt werden.

16. Verfahren nach Anspruch 15,
**dadurch gekennzeichnet,**
**dass** das genetische Material eine zur Wiederherstellung metabolischer Funktionen der verschlossenen Bakterienghosts ausreichende Informationen enthält.

17. Verfahren nach Anspruch 15 oder 16,
**dadurch gekennzeichnet,**
**dass** das genetische Material eine zur Wiederherstellung der Proliferationsfähigkeit der verschlossenen Bakterienghosts ausreichende Information enthält.

18. Verschlossener Bakterienghost erhältlich durch das Verfahren nach einem der Ansprüche 1 bis 17.

19. Verschlossener Bakterienghost nach Anspruch 18,
**dadurch gekennzeichnet,**
**dass** er eine intakte Membran enthält.

20. Verschlossener Bakterienghost nach Anspruch 18 oder 19,
**dadurch gekennzeichnet,**
**dass** er mindestens einen verkapselten Wirkstoff enthält.

21. Verschlossener Bakterienghost nach einem der Ansprüche 18-20,
**dadurch gekennzeichnet,**
**dass** er metabolische Funktionen aufweist.

22. Verschlossener Bakterienghost nach einem der Ansprüche 18-20,
**dadurch gekennzeichnet,**
**dass** er die Fähigkeit zur Proliferation aufweist.

23. Therapeutisches Mittel, umfassend verschlossene Bakterienghosts nach einem der Ansprüche 18-22.

24. Verwendung von verschlossenen Bakterienghosts nach einem der Ansprüche 18-22 zur Herstellung eines Arzneimittels zur Prävention und/oder zur Bekämpfung von durch Pathogene hervorgerufene Erkrankungen, von Tumorerkrankungen oder von Autoimmunerkrankungen.

25. Verwendung nach Anspruch 24 zur Herstellung eines Arzneimittels zur Gentherapie.

26. Verwendung nach Anspruch 24 zur Herstellung eines Arzneimittels als Vakzin.

27. Verwendung nach Anspruch 26, **dadurch gekennzeichnet, dass** das Vakzin ein Nukleinsäurevakzin ist.

28. Diagnostisches Mittel, umfassend verschlossene Bakterienghosts nach einem der Ansprüche 18-22.

29. Verwendung von geschlossenen Bakterienghosts nach einem der Ansprüche 18-22 im nicht-medizinischen Agrarbereich.

30. Verwendung von geschlossenen Bakterienghosts nach einem der Ansprüche 18-22 in der Biotechnologie.

31. Verwendung nach Anspruch 30 als Vehikel zur Gewinnung rekombinanter Polypeptide.

## Claims

1. Method for producing closed bacterial ghosts comprising bringing competent bacterial ghosts into contact with membrane lipid vesicles under conditions under which the electrostatic repulsion between the bacterial ghosts and the lipid vesicles is overcome and under which the membrane is destabilized in the starting materials such that a fusion between the membrane of the bacterial ghosts and the membrane of the membrane lipid vesicles takes place.

2. Method according to claim 1,
**characterized in that**
the ghosts are derived from gram-negative bacteria.

3. Method according to claim 1 or 2,
**characterized in that**
the ghosts are derived from bacteria selected from the group comprising Escherichia coli, Klebsiella, Salmonella, Enterobacter, Pseudomonas, Vibrio, Actinobacillus, Haemophilus, Pasteurella, Bordetella, Helicobacter, Francisella, Brambamella, Erwinia, Pantoea, Streptomyces, Frankia, Serratia, Agrobacterium, Azotobacter, Bradyrhizobium, Burkholderia, Rhizobium, Rhizomonas and Sphingomonas.

4. Method according to one of the previous claims,
**characterized in that**
the ghosts are derived from recombinant bacteria having heterologous membrane polypeptides.

5. Method according to one of the previous claims,
**characterized in that**
the bacterial ghosts are made fusogenic by bringing them into contact with polyvalent metal-cations, in particular calcium and incubation at a temperature of 0 to 5°C.

6. Method according to one of the previous claims,
**characterized in that**
vesicles of homogenized cells, in particular bacterial cells are used as lipid vesicles.

7. Method according to one of the previous claims,
**characterized in that**
liposomes are used as lipid vesicles.

8. Method according to one of the previous claims,
**characterized in that**
membrane-coated viruses are used as lipid vesicles.

9. Method according to one of the previous claims,
**characterized in that**
the fusion between the membrane of the bacterial ghosts and the membrane of the lipid vesicles takes place in the presence of divalent metal cations, in particular calcium ions or/and organic aggregating agents, in particular glycerol, polyethylene glycol or dimethyl sulfoxide.

10. Method according to one of the previous claims,
**characterized in that**
the fusion between the membrane of the bacterial ghosts and the membrane of the lipid vesicles takes place under conditions under which the membranes are in a fluid state.

11. Method according to one of the previous claims,
**characterized in that**
it additionally comprises the packaging of active substances into the bacterial ghosts.

12. Method according to claim 11,
**characterized in that**
the active substances are selected from genetic material, cell components, substances, labelling substances, agriculturally active substances, dyes and combinations thereof.

13. Method according to claim 11 or 12,
**characterized in that**,
the active substances are present in the bacterial ghosts in an immobilized form.

14. Method according to claim 11 or 12,
**characterized in that**
the active substances are present in the bacterial ghosts in a free form.

15. Method according to claim 12 or 14,
**characterized in that**
genetic material and optionally cell components are packaged.

16. Method according to claim 15,
**characterized in that**
the genetic material contains sufficient information to restore metabolic functions of the closed bacterial ghosts.

17. Method according to claim 15 or 16,
**characterized in that**
the genetic material contains sufficient information to restore the ability of the closed bacterial ghosts to proliferate.

18. Closed bacterial ghost obtainable by the method according to one of the claims 1 to 17.

19. Closed bacterial ghost according to claim 18,
**characterized in that**
it contains an intact membrane.

20. Closed bacterial ghost according to claim 18 or 19,
**characterized in that**
it contains at least one encapsulated active substance.

21. Closed bacterial ghost according to one of the claims 18-20,
**characterized in that**
it has metabolic functions.

22. Closed bacterial ghost according to one of the claims 18-20,
**characterized in that**
it has the ability to proliferate.

23. Therapeutic agent comprising closed bacterial ghosts according to one of the claims 18-22.

24. Use of closed bacterial ghosts according to one of the claims 18-22 for producing a pharmaceutical preparation for preventing and/or controlling pathogen-induced diseases, tumour diseases or autoimmune diseases.

25. Use according to claim 24 to produce a pharmaceutical preparation for gene therapy.

26. Use according to claim 24 to produce a pharmaceutical preparation as a vaccine.

27. Use according to claim 26,
**characterized in that**
the vaccine is a nucleic acid vaccine.

28. Diagnostic agent comprising closed bacterial ghosts according to one of the claims 18-22.

29. Use of closed bacterial ghosts according to one of the claims 18-22 in the non-medical agricultural field.

30. Use of closed bacterial ghosts according to one of the claims 18-22 in biotechnology.

31. Use according to claim 30 as a vehicle for obtaining recombinant polypeptides.

## Revendications

1. Procédé de fabrication de fantômes bactériens fermés comportant une étape consistant à mettre en contact les fantômes bactériens appropriés avec une vésicule lipidique à membrane dans des conditions, dans lesquelles la répulsion électrostatique entre les fantômes bactériens et les vésicules lipidiques est surmontée et dans lesquelles la membrane dans les produits de départ est déstabilisée, si bien qu'une fusion se produit entre la membrane du fantôme bactérien et la membrane de la vésicule lipidique à membrane.

2. Procédé selon la revendication 1,
**caractérisé en ce**
**que** les fantômes proviennent de bactéries à Gram négatif.

3. Procédé selon la revendication 1 ou 2,
**caractérisé en ce**
**que** les fantômes proviennent de bactéries choisies parmi le groupe constitué par les *Escherichia coli,* les *Klebsiella,* les *Salmonella,* les *Enterobacter,* les *Pseudomonas,* les *Vibrio,* les *Actinobacillus,* les *Haemophilus,* les *Pasteurella,* les *Bordetella,* les *Helicobacter,* les *Francisella,* les *Brambamella,* les *Erwinia,* les *Pantoea,* les *Streptomyces,* les *Frankia,* les *Serratia,* les *Agrobacterium,* les *Azotobacter,* les *Bradyrhizobium,* les *Burkholderia,* les *Rhizobium,* les *Rhizomonas* et les *Sphingomonas.*

4. Procédé selon l'une quelconque des revendications précédentes,
**caractérisé en ce**
**que** les fantômes sont issus de bactéries recombinantes avec des polypeptides hétérologues membranaires.

5. Procédé selon l'une quelconque des revendications précédentes,
**caractérisé en ce**
**que** les fantômes bactériens sont rendus fusogènes par mise en contact avec des cations métalliques polyvalents, en particulier avec du calcium, et par incubation à une température allant de 0 à 5°C.

6. Procédé selon l'une quelconque des revendications précédentes,
**caractérisé en ce**
**que** des vésicules de cellules homogénéisées, en particulier de cellules bactériennes, sont utilisées comme vésicules lipidiques.

7. Procédé selon l'une quelconque des revendications précédentes,
**caractérisé en ce**
**que** des liposomes sont utilisés comme vésicules lipidiques.

8. Procédé selon l'une quelconque des revendications précédentes,
**caractérisé en ce**
**que** des virus enveloppés d'une membrane sont utilisés comme vésicules lipidiques.

9. Procédé selon l'une quelconque des revendications précédentes,
**caractérisé en ce**
**que** la fusion entre la membrane du fantôme bactérien et la membrane de la vésicule lipidique se produit en présence de cations métalliques bivalents, en particulier d'ions de calcium et/ou d'agents d'agrégation organiques, en particulier de la glycérine, du polyéthylèneglycol ou du diméthylsulfoxyde.

10. Procédé selon l'une quelconque des revendications précédentes,
**caractérisé en ce**
**que** la fusion entre la membrane du fantôme bactérien et la membrane de la vésicule lipidique se produit dans des conditions, dans lesquelles les membranes sont à l'état liquide.

11. Procédé selon l'une quelconque des revendications précédentes,
**caractérisé en ce**
**qu'**il comprend en outre l'étape consistant à envelopper les principes actifs dans les fantômes bactériens.

12. Procédé selon la revendication 11,
**caractérisé en ce**
**que** les principes actifs sont choisis parmi le matériel génétique, les composants cellulaires, les substances, les substances de marquage, les substances actives pour l'agriculture, les colorants ou des combinaisons de ces derniers

13. Procédé selon la revendication 11 ou 12,
**caractérisé en ce**
**que** les principes actifs se trouvent sous une forme immobilisée dans les fantômes bactériens.

14. Procédé selon la revendication 11 ou 12,
**caractérisé en ce**
**que** les principes actifs se trouvent sous une forme libre dans les fantômes bactériens.

15. Procédé selon la revendication 12 ou 14,
**caractérisé en ce**
**que** l'on enveloppe du matériel génétique et éventuellement des composants cellulaires.

16. Procédé selon la revendication 15,
**caractérisé en ce**
**que** le matériel génétique contient des informations suffisantes pour la reconstitution de fonctions métaboliques du fantôme bactérien fermé.

17. Procédé selon la revendication 15 ou 16,
**caractérisé en ce**
**que** le matériel génétique contient des informations suffisantes pour la reconstitution de la capacité de prolifération du fantôme bactérien fermé.

18. Fantôme bactérien fermé pouvant être obtenu par le procédé selon l'une quelconque des revendications 1 à 17.

19. Fantôme bactérien fermé selon la revendication 18,
**caractérisé en ce**
**qu'**il comprend une membrane intacte.

20. Fantôme bactérien fermé selon la revendication 18 ou 19,
**caractérisé en ce**
**qu'**il comprend au moins une substance active encapsulée.

21. Fantôme bactérien fermé selon l'une quelconque des revendications 18-20,
**caractérisé en ce**
**qu'**il présente des fonctions métaboliques.

22. Fantôme bactérien fermé selon l'une quelconque des revendications 18-20,
**caractérisé en ce**
**qu'**il présente une capacité de prolifération.

23. Agent thérapeutique comportant des fantômes bactériens fermés selon l'une quelconque des revendications 18-22.

24. Utilisation de fantômes bactériens fermés selon l'une quelconque des revendications 18-22 pour la fabrication d'un médicament pour la prévention et/ou pour la lutte contre les maladies provoquées par des agents pathogènes, contre les maladies tumorales ou contre les maladies auto-immunes.

25. Utilisation selon la revendication 24 pour la fabrication d'un médicament pour la thérapie génique.

26. Utilisation selon la revendication 24 pour la fabrication d'un médicament comme vaccin.

27. Utilisation selon la revendication 26, **caractérisée en ce que** le vaccin est un vaccin d'acide nucléique.

28. Agent de diagnostique comportant des fantômes bactériens fermés selon l'une quelconque des revendications 18-22.

29. Utilisation de fantômes bactériens fermés selon l'une quelconque des revendications 18-22 dans le domaine agricole non médical.

30. Utilisation de fantômes bactériens fermés selon l'une quelconque des revendications 18-22 en biotechnologie.

31. Utilisation selon la revendication 30 comme vecteur pour obtenir des polypeptides recombinants.
